# EUROPEAN PATENT SPECIFICATION

(11) **EP 1 524 981 B1**
(45) Date of publication and mention of the grant of the patent: **11.03.2009**
(21) Application number: 03766343.2
(22) Date of filing: 28.07.2003
(51) Int. Cl.: A61K 31/53, A61K 9/22, A61K 9/30, A61P 25/08, A61P 25/00, A61P 25/18

(54) **SUSTAINED RELEASE FORMULATIONS COMPRISING LAMOTRIGINE**
ZUSAMMENSETZUNG MIT VERZÖGERTER FREISETZUNG ENTHALTEND LAMOTRIGIN
FORMULATIONS A LIBERATION PROLONGEE CONTENANT DE LA LAMOTRIGINE

(30) Priority: 29.07.2002 GB 0217493; 29.07.2002 GB 0217492; 13.06.2003 GB 0313801
(43) Date of publication of application: 27.04.2005
(73) Proprietor: GLAXO GROUP LIMITED, Greenford, Middlesex UB6 ONN (GB)
(72) Inventor: BUXTON, Ian, Richard, Mississauga, Ontario L5N 6L4 (CA); CURRIE, Robin, Research Triangle Park NC 27709 (US); DELA-CRUZ, Myrna, A., Mississauga, ON L5N 6L4 (CA); GOODSON, Gary, Wayne, Research Triangle Park, NC 27709 (US); KAROLAK, Wlodzimierz, Mississauga, Ontario L5N 6L4 (CA); MALEKI, Mehran, Mississauga, Ontario L5N 6L4 (CA); IYER, Vijay, Mohan, Mississauga, Ontario L5N 6L4 (CA); MUPPIRALA, Gopal, Research Triangle Drive NC, 27709 (US); PARR, Alan Frank, GlaxoSmithKline, North Carolina, 27709 (US); SIDHU, Jagdev, Singh, GlaxoSmithKline, Harlow Essex CM19 5AW (GB); STAGNER, Robert, Allen, GlaxoSmithKline, North Carolina, NC 27709 (US); VIJAY-KUMAR, Akunuri, Venkata, GlaxoSmithKline, Mississauga, Ontario L5N 6L4 (CA)
(74) Representative: Thornley, Rachel Mary
(86) International application number: PCT/EP2003/008368
(87) International publication number: WO 2004/012741

(56) References cited:
- WO-A-97/14415
- WO-A-98/47491
- US-B2- 6 406 716

## Description

This invention relates to novel formulations, in particular tablet formulations.

Lamotrigine, 3,5-diamino-6-(2,3-dichlorophenyl)-1,2,4-triazine is disclosed in US 4,602,017 and EP0021121. Products comprising lamotrigine are marketed under the trade name LAMICTAL^{™} by the GlaxoSmithKline group of companies. Such products are particularly effective for treatment of CNS disorders, particularly epilepsy; pain; oedema; multiple sclerosis and psychiatric indications including bipolar disorder.

Various tablet formulations of lamotrigine have been approved for marketing, for instance, conventional compressed instant release (IR) tablets comprising 25 mg, 50mg, 100 mg, 150 mg or 200 mg of active ingredient. These are administered once, twice or three times daily. For lamotrigine, added to an antiepileptic drug regime containing valproic acid, titration begins at 25 mg every other day for weeks 1 and 2 and increased to 25 mg every day for weeks 3 and 4. After this initial period the maintenance dose of 100 to 400 mg/day can be achieved by increasing the dose by 25 to 50 mg/day. If lamotrigine is added to enzyme-inducing antiepileptic drugs (EIAEDS) without valproic acid the dose is 50 mg/day for weeks 1 and 2 and 100 mg/day in 2 divided doses thereafter. To achieve the maintenance dose of 300 to 500 mg/day in 2 divided doses, doses may be increased by 100 mg/day every 1 to 2 weeks. These regimens provide a therapeutic amount of lamotrigine.

In addition; WO92/13527 (The Wellcome Foundation Limited) describes tablet formulations comprising water dispersible tablets comprising lamotrigine and a dispersing agent where the dispersing agent is a swellable clay such as a smectite and is generally present within the granules of the tablet to provide a tablet which is capable of dispersing in water within 3 minutes to provide a dispersion which will pass through a 710 µm sieve. The tablet can be optionally film coated in which case the dispersion time is less than 5 minutes. Chewable dispersible tablets which may be swallowed whole, chewed or dispersed in a small amount of water are marketed comprising 2mg, 5mg, 25 mg or 100 mg of active ingredient. These are generally administered to paediatric patients.

WO96/17611 (The Wellcome Foundation Limited) discloses pharmaceutical compositions comprising
a) 0.5 to 50% by weight of lamotrigine;
b) from 15 to 50% by weight lactose;
c) from 15 to 50% by weight of starch;
d) from 0.5 to 50% crystalline cellulose; and
e) 5 to 15% by weight of polyvinylpyrrolidone;
and which is in the form of a free flowing powder having the following properties:
(i) no granules having a particle size of greater than 850µm,
(ii) at least 90% by weight having a particle size of 75 to 850 µm,
(iii) the granules disintegrate within 30 minutes according to the Disintegration Test of The Pharmacopoeia of Japan, 12th edition and
(iv) at least 90% by weight of lamotrigine dissolves within 30 minutes when the granules are subjected to the Dissolution Test, method 2 (paddle method) of The Pharmacopoeia of Japan 12th edition 1991.

Lamotrigine is rapidly and completely absorbed after oral administration with negligible first pass metabolism. The absolute bioavailability is about 98%, which is not affected by food.

The chewable dispersible tablets were found to be equivalent to the lamotrigine compressed IR tablets whether they were administered as dispersed in water, chewed and swallowed or swallowed as whole in terms of rate and extent of absorption.

Other drugs available on the market for the treatment of epilepsy are, but not limited to, carbamazepine (Tegretol TM), valproate (Depakote TM), tiagabine (Gabitril TM), levetiracetam (Keppra TM), gabapentin (Neurontin TM) and phenytoin (Dilantin TM). Carbamazepine is available as an instant release tablet, a time releasing chewable tablet (Carbatrol; extended release beads) or Tegretol-XR an osmotic pump tablet, and a liquid to be administered by mouth. Valproate is available as an instant release tablet and a suspension. In the US valproate is also available as Depakote a delayed release (coated) tablet which contains sodium valproate + valproate in 1:1 formulation and also Depakote ER an extended release form). Gabapentin, tiagabine and levetiracetam are available as instant release tablets. Dilantin is available in a 'kapseal' that modifies release.

Existing marketed tablet formulations of lamotrigine provide immediate release of the active ingredients once the tablet reaches the stomach. The peak plasma concentrations occur anywhere from 1.4 to 4.8 hours following drug administration. The disadvantage is that the plasma concentration (pharmacokinetic profile (PK)) achieved with conventional tablets is cyclical, with peaks occurring after administration followed by troughs occurring before the next administration of drug, see Figure (1).

In particular for the treatment of epilepsy it is speculated that the troughs may lead to breakthrough seizures and the peak plasma concentration may result in some adverse events (AE) occurring in some patients or alternatively the rate of increase in plasma concentration in the initial stages before the peak plasma concentration is achieved may also effect the AE profile.

Until recently, it was not known where, in the gastrointestinal tract, lamotrigine is absorbed. In carrying out a regional absorption study it has recently been discovered that the extent of absorption of lamotrigine is consistent when the drug is delivered to any point in the gastrointestinal tract between the stomach and the ascending colon. The extent of absorption is also equivalent whether the drug is delivered as a solid or as a solution.

Accordingly, in a first aspect, the invention comprises a sustained release formulation of lamotrigine or a pharmaceutically acceptable derivative thereof, comprising
1) a core comprising lamotrigine or a pharmaceutically acceptable derivative thereof:
2) an outer coating covering said core, the thickness of said outer coating being adapted such that it is substantially impermeable to the entrance of fluid present in a patient's gastrointestinal tract an environmental fluid and substantially impermeable to the exit of lamotrigine or a pharmaceutically acceptable derivative thereof, and
3) said outer coating including one or more orifices extending from the outside of the coating substantially completely through said coating but not penetrating said core allowing the release of lamotrigine or a pharmaceutically acceptable derivative thereof from the core into enviromental fluidfluid present in a patient's gastrointestinal tract, said orifices having an area or combined area from about 10 to about 60 percent of the face area of said formulation, wherein the release lamotrigine or a pharmaceutically acceptable derivative thereof occurs substantially through said orifice and wherein the outer coat dissolves when the surrounding pH exceeds 5.

When used herein the term "CNS disorder" includes epilepsy; pain; oedema, multiple sclerosis, schizophrenia and psychiatric conditions including bipolar disorder, preferably epilepsy; pain; oedema, and psychiatric conditions including bipolar disorder, particularly epilepsy, pain and bipolar disorder.

When used herein the term "pain" includes acute pain such as musculoskeletal pain, post operative pain and surgical pain, chronic pain such as chronic inflammatory pain (e.g. rheumatoid arthritis and osteoarthritis), neuropathic pain (e.g. post herpetic neuralgia, trigeminal neuralgia, sympathetically maintained pain and pain associated with diabetic neuropathy) and pain associated with cancer and fibromyalgia or pain associated with migraine.

Schizophrenia is a serious psychiatric disease that affects 1% of the world's population. Onset of the disorder occurs typically in the late teens or early 20's and in approximately 80% of cases becomes a lifelong condition. Furthermore, schizophrenia is associated with significant mortality, with 40% of patients attempting suicide within 10 years of the onset of this disorder. The disorder was rated as the 5^{th} leading cause of disability in the US in a joint World Health Organisation - World Bank study in 1996 (Murray and Lopez, 1996).

The clinical presentation of schizophrenia can include positive symptoms, such as hallucinations, delusions, or thought disorder, and negative symptoms such as apathy, avolition, or poverty of speech.

The treatment of schizophrenia relies on the use of anti-dopaminergic drugs following the original discovery in the 1950's of the efficacy and mechanism of action of chlorpromazine. Chlorpromazine and other so-called "typical" antipsychotic drugs are still in common use today, though due to their association with motor side-effects, they are increasingly replaced by the newer "atypical" antipsychotics, such as clozapine (Clozaril^{™}), olanzapine (Zyprexa^{™}) or risperidone (Risperdal^{™}). These newer drugs have a mixed pharmacology which includes dopamine D2 receptor antagonism and antagonism of the 5-HT2a receptor. Despite efficacy and relative safety of these newer drugs, a significant proportion of patients fail to respond to treatment and of those that do, many do not achieve a clinically meaningful improvement in global functioning and quality of life.

In some patients, episodes of maj or depression, mania, or mixed mania can occur alongside symptoms of schizophrenia. The distinction between schizophrenia and mood disorder is then somewhat blurred and a diagnosis of schizoaffective disorder is often used. Treatment of schizoaffective disorder typically requires a combination of an antipsychotic, an antidepressant, a mood stabiliser, and anxiolytic drugs. Although positive psychotic symptoms can usually be controlled, negative symptoms and affective symptoms are poorly treated by current medications.

Despite 40 years of development there remains a significant unmet need for treatment for patients with the chronic debilitating disorder schizophrenia.

Multiple sclerosis (MS) is an autoimmune disease which is a progressive disease of the central nervous system (CNS) in which patches of myelin (the protective covering of nerve fibres) in the brain and spinal cord are destroyed by the body's own immune system. This destruction leads to scarring and damage to the underlying nerve fibres and may manifest itself in a variety of symptoms, depending on the parts of the brain and spinal cord that are affected. Spinal cord damage may result in tingling or numbness as well as heavy and/or weak feeling in the extremities. Damage in the brain may result in muscle weakness, fatigue, unsteady gain, numbness, slurred speech, impaired vision, vertigo and the like. Leandri et al (J Neurol(2000) 247:556-558 reported that lamotrigine had been used in the treatment of trigeminal neuralgia secondary to multiple sclerosis.

When used herein the term "pharmaceutically acceptable derivative" means a salt, ester or salt of such ester which upon administration to the recipient such a human is capable of providing (directly or indirectly) lamotrigine or an active metabolite thereof. Preferred salts are inorganic acid salts such as hydrochloride, hydrobromide, phosphate or organic acid salts such as acetate, fumarate, xinafoate, tartrate, succinate or glutarate.

The term "treatment" as used herein includes the treatment of established disorders and also includes the prophylaxis thereof. This is particularly relevant for epilepsy wherein medication may treat seizures or prevent future seizures from occurring.

As used herein, the term "sustained release" refers to the gradual but continuous release over any extended period of lamotrigine after oral ingestion e.g. 2-20 hours preferably between 6 to 16 hours, and more preferably between 10 and 15 hours, alternatively 10 and 14 hours and which starts when the formulation reaches the stomach and starts to disintegrate/dissolve/erode. The release will continue over a period of time and may continue throughout the small intestine and after the formulation reaches the large intestine.

A further aspect of the invention provides a sustained release formulation of lamotrigine or a pharmaceutically acceptable derivative thereof, wherein substantially all the lamotrigine or a pharmaceutically acceptable derivative thereof is released from the formulation 2 to 20 hours after administration, preferably 6 to 16 hours after administration and more preferably 10 to 15, alternatively 10 to 14 hours after administration.

When used herein "substantially all" means more than 85%, preferably more than 90%. Administration of lamotrigine over this time period delivers it gradually to the sites where lamotrigine is readily absorbed but with a slower rise in serum concentrations and reduced post-dosing peaks to mitigate dosing related adverse events (AE's) yet provide sufficient minimum plasma/serum concentrations (Cmin) to maintain efficacy. A formulation which achieves an area under the curve (AUC) equivalent to the conventional instant/immediate release (IR) tablet (90% confidence interval (CI) for the geometric least squares (GLS) mean ratio should fall within the range 80-125% compared to the reference IR product) is termed "bioequivalent".

Alternatively the sustained release formulation would not be deemed by the Food and Drug Administration (FDA) as bioequivalent to the IR tablets if the points estimate and the associated 90% Confidence Interval for Cmax will not fall within the limit of 80 - 125% relative to the IR product with the AUC remaining within the 80-125% range compared with the reference IR product.

Suitably the formulations are formulated such that the release of the active substances is predominantly in the stomach, small intestine and into the colon.

Preferably the sustained release formulation comprises an amount of lamotrigine or a pharmaceutically acceptable derivative selected from 25mg, 50mg, 100mg, 200mg or 400mg.

Preferably the sustained release formulation is administered in a dosage regimen which is sufficient to maintain control over the disorder.

Preferably the dosage regimen is once a day.

An advantage of sustained release formulations is increased patient compliance.

Socio-economic factors do not influence compliance: non-compliance is just as likely in wealthy, well educated, and healthy patients as it is in patient outside these categories. In most cases, epilepsy is a life-long disease that requires consistent and adequate antiepileptic drug (AED) blood levels to maximize seizure control. Further, it is generally accepted that each additional seizure may increase the risk of recurrence and worsen the overall prognosis. Therefore, primary treatment objectives for patients with epilepsy are maintenance of adequate AED levels and prevention of subsequent seizures. Compliance with the prescribed dosing regimen is essential for the maintenance of therapeutic blood levels.

Patients with epilepsy often are treated with polypharmacy. Patients with severe or refractory epilepsy frequently require the co-administration of two or more AEDs to achieve adequate seizure control. Also, it is not unusual for patients to have other concurrent chronic medical conditions such as depression, heart conditions or diabetes that also require adherence to daily dosing regimens.

The treatment of bipolar disorder is currently recommended as once a day but the present formulation provides a lower rise in plasma concentration of the drug and thereby it is expected that this provides beneficial effects for the patient.

The availability of a once a day tablet for the treatment of pain would be a significant advantage, pain is a continuous disease state, therefore a sustained release formulation would provide pain relief by providing a Cmax at the appropriate point in the day or night depending when the patient's pain is most debilitating.

Preferably the formulation provides about a 10 to 40%, alternatively a 10 to 20% reduction in Cmax over the Cmax obtained in the same patient on an IR dose if administered once daily.

Preferably the formulation provides a time to Cmax (tₘₐₓ) of 8 to 24 hours post dose, alternatively 10 to 16 hours post dose.

Preferably the formulation provides a rate of increase to tₘₐₓ of less than 50% of an individual IR dose.

The formulation may provide at 24 hours post dose a mean minimum serum concentration (Cmin) of at least 80 to 125% compared to the same IR dose in the same patient, or a (Cmin) higher that the IR dose and/or outside the range 80 to 125% compared to the same IR dose.

Preferably the formulation provides a fluctuation index (Cmax-Cmin/Cave) in the range of 0.15 to 0.45.

At present some patients, when administered the conventional IR tablets, experience CNS adverse event (AE's) such as dizziness, ataxia, diplopia and rash.

With the IR formulation the rate of AE's is for example, 31 to 38 % dizziness, 10 to 22% ataxia and 24 to 28 % diplopia. Without wishing to be bound by theory it is believed by the applicants that some of these adverse events relate to peak plasma levels or the rate of increase in plasma concentration after administration and before the peak plasma concentration is achieved. The risk of rash and of serious rash may be related to the initial dose or the rate of dose escalation of lamotrigine, and the development of a formulation that lowers the peak level during titration may lessen the risk of these adverse events.

A further aspect of the invention is the use of lamotrigine or a pharmaceutically acceptable derivative thereof for manufacture of a sustained release formulation in accordance with the invention for the treatment of CNS disorders.

The dosage in a sustained release formulation intended to be swallowed whole where the dosage form integrity is essential for controlling the rate of release may conveniently be provided as a number of swallow tablets or capsules, for instance two, three or four. In cases where the release is achieved from a number of discrete particles, beads or granules, the dosage form need not be swallowed intact as long as the beads or particles themselves remain intact.

The dosage in a sustained release formulation may be also provided as a single tablet.

Preferably, a sustained release formulation of the present invention has an *in vitro* dissolution profile in which 40 to 65%, preferably 45 to 65%, more preferably 45 to 55% of the lamotrigine content is dissolved between 3 to 8 hours, more preferably between 4 to 6 hours; and that 90% of lamotrigine is dissolved between 6 and 16 hours, preferably between 10 to 15 alternatively 10 to 14 hours. In comparison, a conventional, immediate release lamotrigine tablet dissolves 80% within 30 minutes. The dissolution profile may be measured in a standard dissolution assay, for instance <724> Dissolution Test, Apparatus 1 or 2 or 3 or 4, provided in USP 24, 2000 and updated in subsequent supplements, at 37.0 ± 0.5°C, using dilute hydrochloric acid or other suitable media (500-3000 ml) and a rotation speed of 50-100 rpm.

The sustained release formulation may provides an *in vivo* "Area Under the Curve" (AUC) value which is equivalent to that of the existing instant release IR tablet, for instance at least 80%, preferably at least 90% to 110%, more preferably about 100%, but not exceeding 125% of that of the corresponding dosage of lamotrigine taken as a conventional (immediate release) formulation, over the same dosage period, thereby maximising the absorption of lamotrigine from the sustained release formulation.

The pharmacokinetic profile for a dosage of the present invention may be readily determined from a single dosage bioavailability study in human volunteers. Plasma concentrations of lamotrigine may then be readily determined in blood samples taken from patients according to procedures well known and documented in the art.

The person skilled in the art will appreciate that a therapeutically effective amount will depend on the patient's age, size, severity of disease and other medication.

Representative sustained release formulations include a tablet, including swallow tablets, a capsule or granules, typically a swallow tablet, which may be coated.

A further aspect of the invention is a formulation wherein the core further comprises a release retarding excipient, which allows for sustained release of lamotrigine or a pharmaceutically acceptable derivative thereof. Suitable release retarding excipients include release-retarding polymers which may be swellable or not in contact with water or aqueous media such as the stomach contents; polymeric materials which form a gel on contact with water or aqueous media; polymeric materials which have both swelling and gelling characteristics in contact with water or aqueous media and pH sensitive polymers, for instance polymers based upon methacrylic acid copolymers such as the Eudragit (trade mark) polymers, for example Eudragit L (trade mark) which may be used either alone or with a plasticiser.

These sustained release formulations are often referred to in the art, as "matrix formulations" where by the drug is incorporated into a hydrated polymer matrix system and is released via diffusing or erosion, for example WO98/47491 and US 5,242,627.

Release retarding polymers which may be swellable or not include, *inter alia,* cross-linked sodium carboxymethylcellulose, cross-linked hydroxypropylcellulose, hydroxyethylcellulose, high-molecular weight hydroxypropylmethylcellulose, carboxymethylamide, potassium methacrylatedivinylbenzene co-polymer, polymethylmethacrylate, cross-linked polyvinylpyrrolidone, hydroxyethyl cellulose high-molecular weight polyvinylalcohols etc.

Release retarding gellable polymers include methylcellulose, carboxymethylcellulose, low-molecular weight hydroxypropylmethylcellulose, hydroxyethyl cellulose, low-molecular weight polyvinylalcohols, polyoxyethyleneglycols, non-cross linked polyvinylpyrrolidone, xanthan gum etc.

Release retarding polymers simultaneously possessing swelling and gelling properties include medium-viscosity hydroxypropylmethylcellulose and medium-viscosity polyvinylalcohols.

Preferably the release retarding polymer used has a molecular weight in the range 5 to 95 thousand, more preferably in the range 10 to 50 thousand.

A preferred release-retarding polymer is one of the available grades of hydroxypropylmethyl cellulose or hydroxyethyl cellulose.

Examples of polymers which may be used include Methocel K4M (Trade Mark), Methocel E5M (Trade Mark), Methocel E50 (Trade Mark), Methocel E4M (Trade Mark), Methocel E100M (Trade Mark), Methocel K15M (Trade Mark), Methocel K100M (Trade Mark) and Methocel K100LV (Trade Mark), POLYOX WSR N-80 or mixtures thereof. Alternatively examples of polymers which may be used include Methocel K4M (Trade Mark), Methocel E5 (Trade Mark), Methocel E50 (Trade Mark), Methocel E4M (Trade Mark), Methocel K15M (Trade Mark), Methocel K100LV (Trade Mark), POLYOX WSR N-80 or mixtures thereof.

Other known release-retarding polymers which may be incorporated include hydrocolloids such as natural or synthetic gums, cellulose derivatives other than those listed above, carbohydrate-based substances such as acacia, gum tragacanth, locust bean gum, guar gum, agar, pectin, carageenin, soluble and insoluble alginates, carboxypolymethylene, casein, zein, and the like, and proteinaceous substances such as gelatine.

Preferably the release-retarding polymer is Methocel E4M Grade, POLYOX WSR N-80, Methocel K100LV.

The sustained release formulation may also include diluents/compression aid such as lactose, microcrystalline cellulose, dicalcium phosphate, sucrose, mannitol, xylitol; starches, and lubricants such as magnesium stearate, sodium stearyl fumarate and stearic acid. The sustained release formulation may further comprise disintegrants, such as cross-linked polyvinylpyrrolidone (CLPVP) and sodium starch glycollate; binders such as povidone (polyvinylpyrrolidone); flow aids such as silicon dioxide or talc. Typically, the sustained release formulation comprises from about 2.5 to 80% by weight of lamotrigine; from 0 to 70 % by weight of diluent/compression aid and from 0.1 to 2.5 % by weight of lubricant. Preferably the release retarding excipient is a release retarding polymer.

Preferably the release retarding polymer is present in a range of 10 to 70 % by weight polymer.

Preferably the sustained release formulation comprises 2.5 to 80% by weight lamotrigine or a pharmaceutically acceptable derivative thereof.

Preferably the sustained release formulation comprises;
a) 2.5 to 80% by weight lamotrigine or a pharmaceutically acceptable derivative thereof;
b) 10 to 70% by weight release retarding polymer;
c) 0 to 70 % by weight diluent;
d) 0 to 20 % by weight compression aid; and
e) 0.1 to 2.5% by weight lubricants.

In a preferred embodiment the sustained release formulation comprises
a) 2.5 to 80% by weight lamotrigine or a pharmaceutically acceptable derivative thereof;
b) 17.5 to 70% by weight release retarding polymer;
c) 0 to 60 % by weight diluent;
d) 0 to 20 % by weight compression aid; and
e) 0.1 to 2.5% by weight lubricants.

In a preferred embodiment the sustained release formulation the compression aid is absent.

Preferably the sustained release formulation comprises
a) 8.3 to 50 % by weight lamotrigine or a pharmaceutically acceptable derivative thereof;
b) 17.5 to 66.3 % by weight release retarding polymer;
c) 25 to 60 % by weight diluent; and
d) 0.1 to 0.4% by weight lubricant.

More preferably the sustained release formulation comprises
a) 8.3 to 50 % by weight lamotrigine or a pharmaceutically acceptable derivative thereof;
b) 17.5 to 66.3 % by weight Methocel E4MP, CR Grade, POLYOX WSRN-80 or Methocel, K100LV or a mixture thereof;
c) 25 to 60 % by weight lactose; and
d) 0.1 to 0.4% by weight magnesium stearate.

A further aspect of the invention is that there are two phases in the release of lamotrigine or a pharmaceutically acceptable derivative thereof, wherein the release rate in the first phase is different from the release rate in the second phase. Preferably the release rate in the first phase will be slower than the release rate in the second phase. Most preferably in the first phase there is less than 15% release of lamotrigine or a pharmaceutically acceptable derivative thereof in the oesophagus and stomach and the second phase the release of lamotrigine or a pharmaceutically acceptable derivative thereof is at an increased rate than the first phase.

For example the first phase would be a period of on average 0 to 2 hours, and the second phase is 2 to 20 hours, preferably 2 to 16 hours, preferably 2 to 15 hours. It will be appreciated that in every patient the gastrointestinal timings can differ and therefore the 2 hours is an average across the patient population.

Preferably there is less than 10% release of lamotrigine of a pharmaceutically acceptable derivative thereof in the first phase.

This aspect of the invention is particularly advantageous as it reduces the release of lamotrigine in the stomach where the lamotrigine solubility is higher (compared to lower regions of the gastro-intestinal tract). It may produce a substantially linear increase in plasma lamotrigine concentrations *in vivo*.

The invention is a sustained release formulation comprising;
1) a core comprising lamotrigine or a pharmaceutically acceptable derivative thereof:
2) an outer coating covering said core, the thickness of said outer coating being adapted such that it is substantially impermeable to the entrance of fluid present in a patient's gastrointestinal tract and substantially impermeable to the exit of lamotrigine or a pharmaceutically acceptable derivative thereof, and
3) said outer coating including one or more orifices extending from the outside of the coating substantially completely through said coating but not penetrating said core allowing the release of lamotrigine or a pharmaceutically acceptable derivative thereof from the core into fluid present in a patient's gastrointestinal tract, said orifices having an area or combined area from about 10 to about 60 percent of the face area of said formulation, wherein the release lamotrigine or a pharmaceutically acceptable derivative thereof occurs substantially through said orifice and wherein the outer coat dissolves when the surrounding pH exceeds 5.

Preferably the core further comprises a release retarding excipient. More preferably the release retarding excipients are as described above for the matrix formulations.

Furthermore the outer coat may dissolve by 0.3 to 5 hours after administration or when the surrounding pH exceeds 5 preferably 5.5.

More preferably the core further comprises a release retarding excipient and the outer coat dissolves by 0.3 to 5 hours after administration or when the surrounding pH exceeds 5 preferably 5.5.

Preferably the release retarding excipient is as described above for matrix formulation.

Preferably the thickness of the outer coating is in the range 0.05 mm to 0.30 mm, preferably 0.10mm to 0.20mm.

Preferably the outer coat includes one or two orifices.

Preferable the outer coating is selected from the group consisting of ethyl cellulose, acrylate polymers, polyamides, polymethacrylates, waxes, polyanhydrides, polyglycolides, polyactides, polybutyrates, polyvalerates, polycaprolactones, natural oils, polydimethylsiloxane, cross-linked or uncrossed linked sodium carboxymethylcellulose starch, polyvinylpyrollidone, cellulose ethers, cellulose acetate phthalate, polyvinylalcohol phthalate, shellac, zein, hydroxypropylmethyl cellulose phthalate, methacrylic acid polymers or copolymers, one or more of the above and the like.

Preferably the formulation comprises; 2.5 to 80% by weight lamotrigine or a pharmaceutically acceptable derivative thereof.

In a preferred embodiment the sustained release formulation comprises a core comprising
a) 2.5 to 80% by weight lamotrigine or a pharmaceutically acceptable derivative thereof;
b) 17.5 to 70% by weight release retarding polymer;
c) 0 to 60 % by weight diluent;
d) 0 to 20 % by weight compression aid ; and
e) 0.1 to 2.5% by weight lubricants and
   an outer coat comprising
f) 0.05 mm to 0.30 mm of polymer;

In a preferred embodiment of the sustained release formulation, the compression aid is absent.

In a more preferred embodiment the sustained release formulation comprises a core comprising
a) 5 to 66% by weight lamotrigine or a pharmaceutically acceptable derivative thereof;
b) 17.5 to 66.3% by weight release retarding polymer;
c) 0 to 60 % by weight diluent; and
d) 0.1 to 0.4% by weight lubricants;
   and an outer coat comprising
e) 0.05 mm to 0.30 mm of polymer.

Preferably the release retarding polymer is a HPMC polymer, more preferably it is selected from Methocel E4M, CR Grade, POLYOX WSRN-80 or Methocel K100LV, or a mixture thereof.

Preferably the outer coat polymer is a methacrylic acid copolymer more preferably Eudragit.

Preferably the lamotrigine or a pharmaceutically acceptable derivative thereof is present in an amount 5 to 55%.

More preferably the sustained release formulation comprises a core comprising:
a) 5 to 55 % by weight lamotrigine or a pharmaceutically acceptable derivative thereof;
b) 17.5 to 66.3 % by weight Methocel E4MP, CR Grade, POLYOX WSRN-80 or Methocel, K100LV or a mixture thereof;
c) 25 to 60 % by weight lactose; and
d) 0.1 to 0.4% by weight magnesium stearate;
   and an outer coat comprising
e) 0.05mm to 0.30mm of Eudragit L30.

"Environmental fluid" means the fluid present or mimic the dissolution properties of that in a patient's gastrointestinal tract.

"Dispensing period" means from the time of administration to the end of the release of lamotrigine or the pharmaceutically acceptable derivative thereof e.g. 0 to 20 hours, preferably 0 to 16 hours, more preferably 0 to 15 alternatively, 0 to 14 hours.

When used herein in "substantially impermeable" means that little or no lamotrigine or a pharmaceutically acceptable derivative thereof is allowed to egress through the coat e.g. less than less than 5%, preferably less than 2% even more preferably less than 1% or that little or no enviromental fluid is allowed to ingress through the coat e.g. less than less than 5%, preferably less than 2% even more preferably less than 1%

When used herein the term "orifice" means an aperture in the outer coat, for example an opening in the outer coat of the tablet and include a portion of the surface of the outer coat which is significantly thinner that the remainder of the coat for example.

When used herein the term "release" means, the exiting of lamotrigine or a pharmaceutically acceptable derivative thereof from the formulation into environmental fluid for example by dissolution, diffusion, osmosis or erosion.

Matrix tablets as described above can be compression or spray coated with an aqueous solution of polymer to produce a film coat. Coating can take place in any standard coating machine known to the person skilled in the art, for example a Vector^{™} machine. The orifice or orifices are then drilled into the tablet film coat. The orifices can be produced by removing certain portion(s) of the film coat from the previously coated tablet surface.

Typically the surface area removed is between 0.1% to 50%, preferably around 15-20%. The orifices can be produced by mechanical drilling, ultrasonic cutting or laser, mechanical drilling is preferred.

The orifices can be any shape, for example oval, round, square or even shaped as text, for example a company logo, preferably the orifice is round

The orifice size will depend on the size of the tablet but for example can be 0.1 to 6-7 mm for 9-10 mm tablet, preferably 4-4.5 mm.

If the tablet has more than one orifice, the orifices can be on the same or difference faces of the tablets, preferably on opposite faces.

The orifice can be centred on the face of the tablet or off centre.

Tablets may be round, oval, elliptical, shield or capsule shape, shallow to deep convex. Preferably the tablet is round or oval shaped, standard convex.

Tablet formulations of the invention may contain a waxy or similar water insoluble material in order to form the matrix. Such a tablet may be formed by dry blending the drug and any diluent materials with the waxy material in particulate form. Examples of suitable waxy materials are cetyl alcohol, stearyl alcohol, palmitoyl, alcohol, oleyl alcohol and carnuba wax. There resulting blend is then compressed into tablets using conventional tablet making technologies. An alternative methods of manufacturing these tablets would be to granulate the drug with the diluent materials with a suitable volatile granulating fluid (water, ethanol, isopropanol) and to dry the granules, then coat them with a molten waxy material. The resultant granules are then compressed into tablet using conventional tablet making technology.

Granule based tablets can also be made by spraying a solution or suspension of one of the methacrylate based release controlling agents (Eudragit - trade mark) onto a blend of the drug mixed with one of the common diluents. Examples of suitable Eudragits are NE30D, L, S. The granules formed in the process are then dried and compressed using conventional tablet making technology.

The tablet formulations of the invention may be wholly or partly covered by a coating layer, which may be a protective layer to prevent ingress of moisture or damage to the tablet. The protective layer may itself contain active material content, and may, for example, be an immediate release layer, which immediately disintegrates in contact with water or aqueous media. Preferred materials for the protective layer are hydroxypropylmethylcellulose and polyethylene glycol, with titanium dioxide as an opacifying agent, for instance as described in WO 95/28927 (SmithKline Beecham).

As well as active material content etc, the tablet of the invention may also include a pH modifying agent, such as a pH buffer. A suitable buffer is calcium hydrogen phosphate.

The protective layer, if present, may typically be made up by a wet granulation technique, or by dry granulation techniques such as roller compaction. Typically the protective layer material, e.g. Methocel (trade mark) is suspended in a solvent such as ethanol containing a granulation acid such as Ethocel or Polyvidon K-30 (trade mark), followed by mixing, sieving and granulation. Typically a first layer may be formed, then a barrier layer deposited upon it, e.g. by compression, spraying or immersion techniques, then the second layer may be formed so that the barrier layer is sandwiched between the first and second layers. Additionally, or alternatively, the first and second layers may be formed and a barrier layer may then be formed, for instance by compression, spraying or immersion, on one or more of the end faces of the tablet.

Capsules according to the present invention comprise, in addition to the drug substance, excipients typically included in a capsule, for instance starch, lactose, microcrystalline cellulose, ethyl cellulose, magnesium stearate. Preferably, capsules are prepared from materials such as HPMC or a gelatine/PEG combination. Preferably the capsules will contain beads or granules. These beads or granules are composed of the drug substance in a concentration of between 5% and 95%, preferably 20 to 80%, most preferably 50 to 80%. The drug substance is mixed with a suitable granulating aid such as microcrystalline cellulose, lactose, and granulated using a suitable granulating fluid such as water, ethanol and/or isopropanol. The wet granules are forced through small orifices of 0.5mm to 3 mm diameter then spheronised into discrete particles using a rapidly spinning disc. The spherical particles are then dried and coated with a release controlling film coat containing for example ethyl cellulose, pH sensitive or insensitive methacrylic acid copolymers and their derivatives. The coated particles are filled into suitable capsule shells.

Preferably, the unit dosage forms of the present invention are packaged in containers that inhibit the ingress of atmospheric moisture, for instance blister packs, tightly closed bottles or desiccated pouch packs etc which are conventional in the art. Preferred bottles include HDPE bottles.

Other sustained release formulations which may be suitable for incorporating lamotrigine or other suitable derivatives thereof are described in:
Sustained Release Medications, Chemical Technology Review No. 177. Ed. J.C. Johnson. Noyes Data Corporation 1980.
Controlled Drug Delivery, Fundamentals and Applications, 2nd Edition. Eds. J.R. Robinson, V.H.L. Lee. Mercel Dekkes Inc. New York 1987.

Examples of delayed release formulations which are suitable for incorporating lamotrigine or other suitable derivatives thereof are described in:
Remington's Pharmaceutical Sciences 16th Edition, Mack Publishing Company 1980, Ed. A. Osol.

A further aspect of the invention is a sustained release formulation of the invention additionally containing a second active ingredient selected from carbamazepine, valproic acid, gabapentin, diazepam, phenytoin, bupropion or paroxetine HCl.

Preferably both the lamotrigine and the second active ingredient are both administered in a sustained release fashion. Alternatively the formulation contains 2 phases, one sustained release phase comprising lamotrigine and a second instant release phase comprising the second active ingredient.

The invention will now be described by way of example only, with reference to the accompanying drawings, in which:

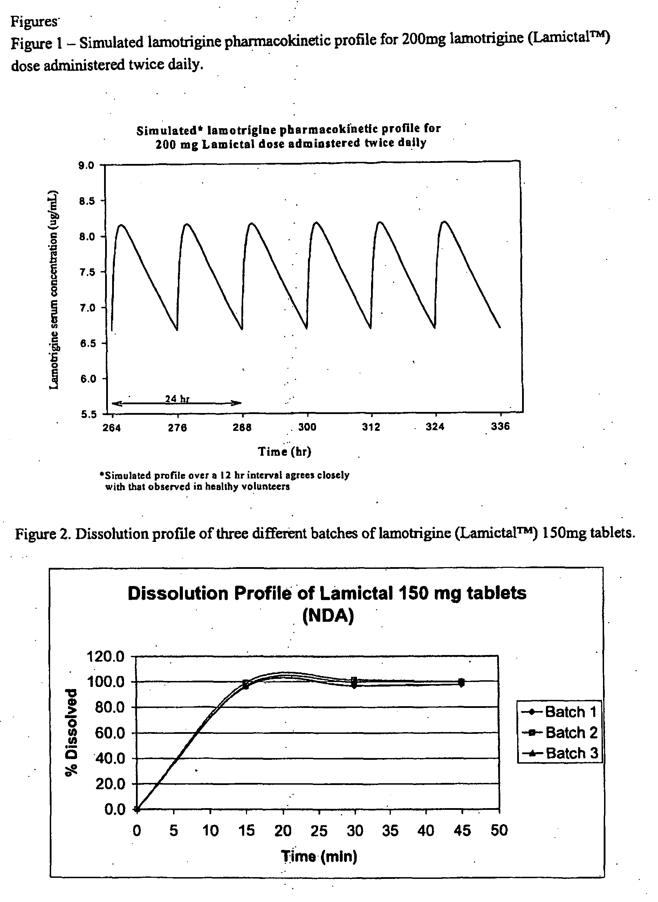

Figure 1 Simulated lamotrigine pharmacokinetic profile for 200mg lamotrigine IR tablets administered twice daily.

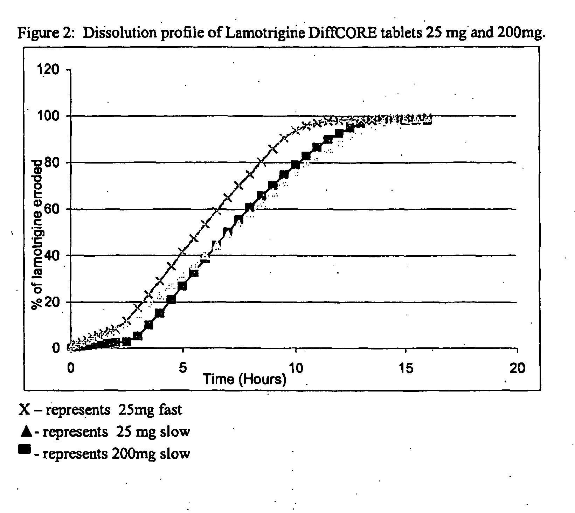

Figure 2. Dissolution profile of lamotrigine DiffCORE tablets 25mg and 200mg of Example 4.

The present invention also extends to formulations which are bioequivalent to the tablets or formulations of the present invention, in terms of both rate and extent of absorption, for instance as defined by the US Food and Drug Administration and discussed in the so-called "Orange Book" (Approved Drug Products with Therapeutic Equivalence Evaluations, US Dept of Health and Human Services, 19th edn, 1999).

All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

### Example 1: DiffCORE tablets

### Formulation Details for DiffCORE Tablets, 25mg

| **Ingredients** | **Quantity (mg/tablet)¹** | |
|---|---|---|
| | **Fast** | **Slow** |
| CORE | | |
| Lamotrigine | 25 | 25 |
| Hydroxypropyl Methylcellulose (HPMC), K100LV, Prem CR USP/EP | 37.8 | 53.55 |
| Hydroxypropyl Methylcellulose (HPMC), E4M, Prem CR, USP | 52.2 | 73.95 |
| Lactose monohydrate, 200 mesh NF | 183.8 | 146.31 |
| Purified Water, EP/USP | | |
| Magnesium Stearate, EP/NF | 1.2 | 1.2 |

| OUTER COAT | | |
|---|---|---|
| Eudragit L30 D-55 (30%w/w solution) | 13.08 | 12.98 |
| Red Iron Oxide, USP | 0.15 | 0.278 |
| Triethyl Citrate, NF | 1.37 | 1.36 |
| Glyceryl Monostearate, NF | 0.37 | 0.37 |
| Polysorbate 80, NF | 0.016 | 0.016 |
| Purified Water EP/USP | | |

| | | |
|---|---|---|
| Fast represents a release period of 12 hours and slow represents a release period of 15 hours. | | |

### Formulation Example of DiffCORE Tablets, 200mg

| **Ingredients** | **Quantity (mg/tablet)¹** |
|---|---|
| | **Slow** |
| CORE | |
| Lamotrigine | 200 |
| Hydroxypropyl Methylcellulose (HPMC), K100LV, Prem CR USP/EP | 62.64 |
| Hydroxypropyl Methylcellulose (HPMC), E4M, Prem CR, USP | 45.36 |
| Lactose monohydrate, 200 mesh NF | 90.4 |
| Purified Water, EP/USP | |
| Magnesium Stearate, EP/NF | 1.6 |

| OUTER COAT | |
|---|---|
| Eudragit L30 D-55 (30%w/w solution) | 17.3 |
| Red Iron Oxide, USP | 0.37 |
| Triethyl Citrate, NF | 1.81 |
| Glyceryl Monostearate, NF | 0.494 |
| Polysorbate 80, NF | 0.02 |
| Purified Water EP/USP | |

| | |
|---|---|
| The core of the tablets were prepared as described in Example 2 and subsequently coated. | |

### Coasting.

The tablets were film-coated using a standard coating machine e.g. a Vector^{™} machine purchased from Vector, or equivalent coater. Tablets were sprayed with an aqueous solution of Eudragit 10% w/w as described below. Tablets were coated up to a 5% theoretical weight gain.

### Example of 10% Coating Solution Preparation

### Part A

350 ml Eudragit L30 D55 30% solution was diluted with 150 ml of water. 11 g of Triethyl Citrate was added and the solution mixed thoroughly.

### Part B

440 g of purified water was added to a separate vessel and heated to approximately 60C. Using appropriate mixer (high shear) for Kalish mixture 0.13 g of Polysorbate 80 NF and 3.0 g of Glyceryl Monostearate, NF were incorporated into solution. 4.0 g of iron oxide was added and homogenised for 15 minute at high speed.

Part A and Part B were combined and the final weight adjusted with purified water to 1000 g and mixed.

### Drilling

The tablets were drilled mechanically using a standard drill press. A tablet was placed in a tablet holder and carefully drilled until the film coat was removed from the drilled surface, then the tablet was flipped over and the opposite side subsequently drilled. Upon completion the drilled tablet was inspected for weight loss (orifice depth), quality of the orifice edge and overall appearance.

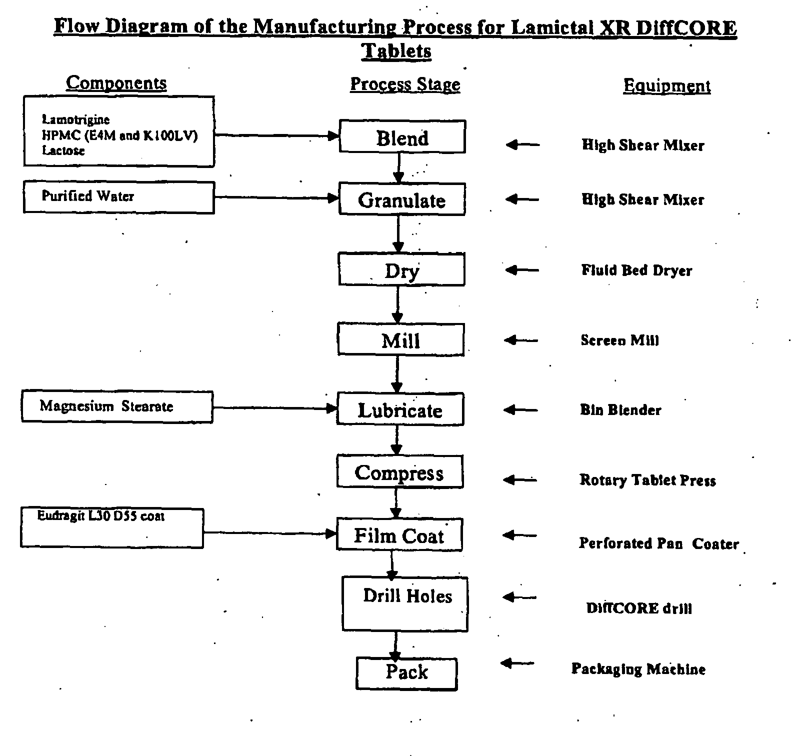

| Tradename | Generic description | Supplier |
|---|---|---|
| Methocel E4M | hydroxypropyl methylcellulose with 28-30% methoxyl and 8.5% hydroxypropyl substitution, having a 4,000 mPa s nominal viscosity (2% solution in water) | Dow |
| Methocel K15M | hydroxypropyl methylcellulose with 22% methoxyl and 8.1 % hydroxypropyl substitution, having a 15,000 mPa s nominal viscosity (2% solution in water) | Dow |
| Methocel K100M | hydroxypropyl methylcellulose with 22% methoxyl and 8.1 % hydroxypropyl substitution, having a 100,000 mPa s nominal viscosity (2% solution in water) | Dow |
| Methocel K4M | hydroxypropyl methylcellulose with 22% methoxyl and 8.1 % hydroxypropyl substitution, having a 4,000 mPa s nominal viscosity (2% solution in water) | Dow |
| Methocel E5 | hydroxypropyl methylcellulose with 29% methoxyl and 8.5% hydroxypropyl substitution, having a 5 mPa s nominal viscosity (2% solution in water) | Dow |
| Methocel E5M | hydroxypropyl methylcellulose with 29% methoxyl and 8.5% hydroxypropyl substitution, having a 5,000 mPa s nominal viscosity (2% solution in water) | Dow |
| Methocel E50 | hydroxypropyl methylcellulose with 29% methoxyl and 8.5% hydroxypropyl substitution, having a 50 mPa s nominal viscosity (2% solution in water) | Dow |
| Methocel K100LV | low viscosity Hydroxypropyl Methylcellulose | Dow |
| POLYOX^{™} WSRN-80 | high molecular weight water-soluble poly (ethylene oxide) polymer Molecular weight of 200,000, having a nominal viscosity of 55-90 cP (5% solution). | Dow |
| Opadry (YS-2-7013) | hydroxypropylmethylcellulose aqueous dispersion | Colorcon |
| Surelease (E-7-19010) | aqueous ethylcellulose dispersion | Colorcon |
| Eudragit® L30D-55 | methacrylic acid-ethyl acrylate copolymer | Rohm Pharma |
| Eudragit® RS 30D | | Rohm Pharma |
| Eudragit® RL 30D | ammonium-methacrylic copolymer RL=10% quat. ammonium RS=5% quat. ammonium | |
| Aquacoat | ethylcellulose latex suspension | (FMC) |

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation the following claims:

## Claims

1. A sustained release formulation comprising
1) a core comprising lamotrigine or a pharmaceutically acceptable derivative thereof,
2) an outer coating covering said core, the thickness of said outer coating being adapted such that it is substantially impermeable to the entrance of fluid present in a patient's gastrointestinal tract and substantially impermeable to the exit of lamotrigine or a pharmaceutically acceptable derivative thereof, and
3) said outer coating including one or more orifices extending from the outside of the coating substantially completely through said coating but not penetrating said core allowing the release of lamotrigine or a pharmaceutically acceptable derivative thereof from the core into fluid present in a patient's gastrointestinal tract, said orifices having an area or combined area from about 10 to about 60 percent of the face area of said formulation,
wherein the release lamotrigine or a pharmaceutically acceptable derivative thereof occurs substantially through said orifice and wherein the outer coat dissolves when the surrounding pH exceeds 5.

2. A sustained release formulation as claimed in claim 1 wherein the core further comprises a release retarding excipient.

3. A sustained release formulation as claimed in claim 1 or claim 2 wherein the formulation comprises a core comprising
a) 2.5 to 80% by weight lamotrigine or a pharmaceutically acceptable derivative thereof;
b) 17.5 to 70% by weight release retarding polymer;
c) 0 to 60 % by weight diluent;
d) 0 to 20 % by weight compression aid; and
e) 0.1 to 2-5% by weight lubricants and
an outer coat comprising
f) 0.05 mm to 0.30 mm of polymer.

4. A sustained release formulation as claimed in any one of claims 1 to 3, wherein the release retarding excipient or release retarding polymer is a HPMC polymer.

5. A sustained release formulation as claimed in any one of claims 1 to 4 wherein the outer coat comprises a methacrylic acid copolymer.

6. A sustained release formulation as claimed in any one of claims 1 to 5 which upon administration to a human produce AUC values outside the range 80 to 125% and a Cₘₐₓ being of about 30% less than an instant release tablet containing the same amount of lamotrigine or a pharmaceutically acceptable derivative thereof.

## Patentansprüche

1. Formulierung mit verlängerter Wirkstofffreisetzung umfassend
1) einen Kern, umfassend Lamotrigin oder ein pharmazeutisch verträgliches Derivat davon,
2) eine äußere Beschichtung, die den Kern bedeckt, wobei die Dicke der äußeren Beschichtung so angepasst ist, dass sie im Wesentlichen nicht permeabel ist für den Eintritt von Flüssigkeiten, die im Magen-Darm-Trakt eines Patienten vorhanden sind, und im Wesentlichen nicht permeabel ist für den Austritt von Lamotrigin oder eines pharmazeutisch verträglichen Derivats davon, und
3) wobei die äußere Beschichtung eine oder mehr Öffnungen enthält, die von der Außenseite der Beschichtung im Wesentlichen komplett durch die Beschichtung reichen, aber den Kern nicht penetrieren, und die die Freisetzung von Lamotrigin oder eines pharmazeutisch verträglichen Derivats davon aus dem Kern in Flüssigkeit, die im Magen-Darm-Trakt eines Patienten vorkommt, zulässt, wobei die Öffnungen einen Bereich oder einen zusammengefassten Bereich von etwa 10 bis etwa 60% der Oberfläche der Formulierung haben,
wobei die Freisetzung von Lamotrigin oder eines pharmazeutisch verträglichen Derivats davon im Wesentlichen durch die Öffnungen stattfindet und wobei sich die äußere Beschichtung auflöst, wenn der Umgebungs-pH den Wert 5 überschreitet.

2. Formulierung mit verlängerter Wirkstofffreisetzung nach Anspruch 1, wobei der Kern des Weiteren einen freisetzungsverzögernden Exzipienten umfasst.

3. Formulierung mit verlängerter Wirkstofffreisetzung nach Anspruch 1 oder 2, wobei die Formulierung einen Kern umfasst, umfassend
a) 2,5 bis 80 Gew.-% Lamotrigin oder ein pharmazeutisch verträgliches Derivat davon;
b) 17,5 bis 70 Gew.-% freisetzungsverzögendes Polymer;
c) 0 bis 60 Gew.-% Streckmittel;
d) 0 bis 20 Gew.-% Kompressionshilfe; und
e) 0,1 bis 2,5 Gew.-% Gleitmittel
und eine äußere Beschichtung, umfassend
f) 0,05 mm bis 0,30 mm Polymer.

4. Formulierung mit verlängerter Wirkstofffreisetzung nach einem der Ansprüche 1 bis 3, wobei der freisetzungsverzögernde Exzipient oder das freisetzungsverzögernde Polymer ein HPMC-Polymer ist.

5. Formulierung mit verlängerter Wirkstofffreisetzung nach einem der Ansprüche 1 bis 4, wobei die äußere Beschichtung ein Methacrylsäure-Copolymer umfasst.

6. Formulierung mit verlängerter Wirkstofffreisetzung nach einem der Ansprüche 1 bis 5, welche nach Verabreichung an einen Menschen AUC-Werte außerhalb des Bereiches von 80 bis 125 % hervorruft und einen Cₘₐₓ-Wert von etwa 30% weniger als eine Tablette mit sofortiger Wirkstofffreisetzung aufweist, die dieselbe Menge an Lamotrigin oder eines pharmazeutisch verträglichen Derivats davon beinhaltet.

## Revendications

1. Formulation à libération prolongée, comprenant
1) un noyau comprenant de la lamotrigine ou un de ses dérivés pharmaceutiquement acceptables,
2) un enrobage extérieur recouvrant ledit noyau, l'épaisseur dudit enrobage extérieur étant adaptée de telle sorte que cet enrobage soit substantiellement imperméable à la pénétration du fluide présent dans le tractus gastro-intestinal d'un patient et substantiellement imperméable à la sortie de la lamotrigine ou d'un de ses dérivés pharmaceutiquement acceptables ; et
3) ledit enrobage extérieur comprenant un ou plusieurs orifices s'étendant de l'extérieur de l'enrobage pratiquement totalement à travers ledit enrobage mais ne pénétrant pas dans ledit noyau, ce qui permet la libération de la lamotrigine ou d'un de ses dérivés pharmaceutiquement acceptables du noyau par passage dans le fluide présent dans le tractus gastro-intestinal d'un patient, lesdits orifices ayant une surface ou surface combinée d'environ 10 à environ 60 % de la surface faciale de ladite formulation,
dans laquelle la libération de la lamotrigine ou d'un de ses dérivés pharmaceutiquement acceptables se produit substantiellement à travers ledit orifice et dans laquelle l'enrobage extérieur de dissout lorsque le pH environnant est supérieur à 5.

2. Formulation à libération prolongée suivant la revendication 1, dans laquelle le noyau comprend en outre un excipient retardant la libération.

3. Formulation à libération prolongée suivant la revendication 1 ou la revendication 2, qui comprend un noyau comprenant :
a) 2,5 à 80 % en poids de lamotrigine ou d'un de ses dérivés pharmaceutiquement acceptables ;
b) 17,5 à 70 % en poids d'un polymère retardant la libération ;
c) 0 à 60 % en poids d'un diluant ;
d) 0 à 20 % en poids d'un auxiliaire de compression ; et
e) 0,1 à 2,5 % en poids de lubrifiants et
un enrobage extérieur comprenant
f) 0,05 mm à 0,30 mm d'un polymère.

4. Formulation à libération prolongée suivant l'une quelconque des revendications 1 à 3, dans laquelle l'excipient retardant la libération ou le polymère retardant la libération est un polymère HPMC.

5. Formulation à libération prolongée suivant l'une quelconque des revendications 1 à 4, dans laquelle l'enrobage extérieur comprend un copolymère d'acide méthacrylique.

6. Formulation à libération prolongée suivant l'une quelconque des revendications 1 à 5, qui, par administration à un être humain, produit des valeurs de AUC à l'extérieur de l'intervalle de 80 à 125 % et une Cₘₐₓ qui est inférieure d'environ 30 % à celle d'un comprimé à libération instantanée contenant la même quantité de lamotrigine ou d'un de ses dérivés pharmaceutiquement acceptables.
